(19) Europäisches Patentamt
European Patent Office
Office européen des brevets

(11) Veröffentlichungsnummer: **0 202 461**
**A1**

# (12) EUROPÄISCHE PATENTANMELDUNG

(21) Anmeldenummer: **86105042.5**

(22) Anmeldetag: **12.04.86**

(51) Int. Cl.[4]: **C 07 D 233/50**
**A 61 K 31/415**

(30) Priorität: **20.04.85 DE 3514351**

(43) Veröffentlichungstag der Anmeldung:
**26.11.86 Patentblatt 86/48**

(84) Benannte Vertragsstaaten:
**AT BE CH DE FR GB IT LI LU NL SE**

(71) Anmelder: **BOEHRINGER INGELHEIM KG**
**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**
**D-6507 Ingelheim am Rhein(DE)**

(84) Benannte Vertragsstaaten:
**GB**

(72) Erfinder: **Esser, Franz, Dr.**
**Posener Strasse 30**
**D-6507 Ingelheim am Rhein(DE)**

(72) Erfinder: **Stähle, Helmut, Dr.**
**Rotweinstrasse 23**
**D-6507 Ingelheim am Rhein(DE)**

(72) Erfinder: **Köppe, Herbert, Dr.**
**Neuweg 72**
**D-6507 Ingelheim am Rhein(DE)**

(72) Erfinder: **Abele, Wolfgang, Dr.**
**Frankenstrasse 50**
**D-6531 Waldalgesheim(DE)**

(72) Erfinder: **Pichler, Ludwig, Dr.**
**Gusshausstrasse 24/II**
**A-1040 Wien(AT)**

(72) Erfinder: **Kobinger, Walter, Prof.**
**Belghofergasse 27**
**A-1120 Wien(AT)**

(72) Erfinder: **Arndts, Dietrich, Dr.**
**Mühlstrasse 7**
**D-6531 Appenheim(DE)**

(54) **Neue substituierte 2-(N-Alkinyl-N-phenylamino)imidazolinderivate, deren Säureadditionssalze, diese enthaltende Arzneimittel und Verfahren zu deren Herstellung.**

(57) Die Erfindung betrifft neue substituierte 2-(N-Alkinyl-N-phenylamino) imidazolinderivate der allgemeinen Formel I

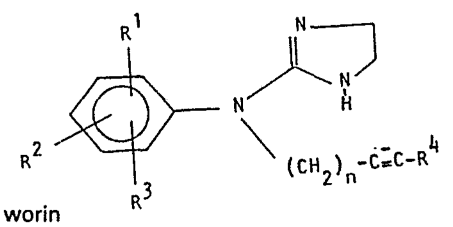

worin
$R^1$, $R^2$, $R^3$ und $R^4$ wie in der Beschreibung definiert sind, n die Zahlen 1, 2 oder 3 bedeuten können sowie deren Säureadditionssalze.

Die neuen Verbindungen besitzen wertvolle pharmakologische und therapeutische Eigenschaften.

Die neuen Verbindungen erhält man durch selektive Alkinylierung am exocyclischen Stickstoffatom der entsprechenden Phenyliminoimidazolidine der allgemeinen Formel II.

EP 0 202 461 A1

2-Phenylaminoimidazoline beanspruchen wegen ihrer außerge-wöhnlichen pharmakologischen und therapeutischen Eigenschaften seit langem ein starkes Interesse.

Gegenstand der Erfindung sind neue substituierte 2-(N-Alkinyl-N-phenylamino)imidazolinderivate der allgemeinen Formel I

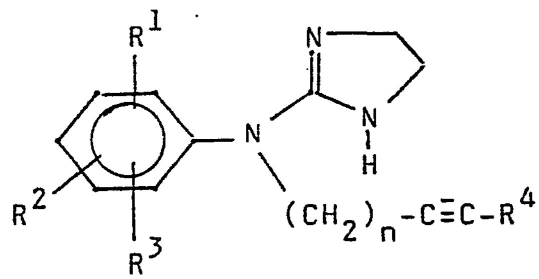

worin $R^1$, $R^2$ und $R^3$ gleich oder verschieden sein können und Wasserstoff, Halogen, eine verzweigte oder unverzweigte niedere Alkyl-, Halogenalkyl-, Alkoxy- oder Halogenalkoxygruppe, eine Cycloalkylgruppe;
$R^4$ Halogen, eine verzweigte oder unverzweigte niedere Alkylgruppe, eine Arylgruppe, gegebenenfalls mit Halogen oder wenigstens einer verzweigten oder unverzweigten niederen Alkyl-, Halogenalkyl-, Alkoxy- oder Halogenalkoxygruppe substituiert, oder eine Aralkylgruppe, gegebenenfalls mit Halogen oder wenigstens einer verzweigten oder unverzweigten niederen Alkyl-, Halogenalkyl-, Alkoxy- oder Halogenalkoxygruppe substituiert,
und n die Zahlen 1, 2 oder 3 bedeuten können sowie deren Säureadditionssalze.

In einer bevorzugten Ausführungsform der allgemeinen Formel I werden unter verzweigten oder unverzweigten niederen Alkyl-, Halogenalkyl-, Alkoxy- und Halogenalkoxygruppen Gruppen mit 1 bis 4 Kohlenstoffatomen verstanden, die sich beispielsweise von einer Methyl-, Ethyl-, Propyl-, Isopropyl-oder t-Butylgruppe ableiten lassen. Die bevorzugte Arylgruppe ist Phenyl, die bevorzugte Aralkylgruppe ist eine Phenalkylgruppe wie z.B. die Benzylgruppe. Halogen bedeutet soweit nicht anderes angegeben, eines der Atome Fluor, Chlor, Brom oder Jod.

Besonders bevorzugt sind Verbindungen der allgemeinen Formel I, in denen
$R^1$, $R^2$ und $R^3$ gleich oder verschieden sind und Wasserstoff, Fluor, Chlor, Methyl, Cyclopropyl, Trifluormethyl, Methoxy oder Trifluormethoxy;
$R^4$ ein Halogenatom, bevorzugt Brom, eine Methyl- oder Phenylgruppe und n die Zahlen 1 oder 2 bedeuten können, wie auch deren Säureadditionssalze.

Zu diesen besonders bevorzugten Verbindungen zählen:

2-[N-2-Butinyl-N-(2,6-dichlorphenyl)amino]imidazolin;

2-[N-2-Butinyl-N-(2-trifluormethoxy-5-fluorphenyl)amino]-imidazolin;

2-[N-2-Butinyl-N-(2-trifluormethyl-6-fluorphenyl)amino]-imidazolin;

2-[N-2-Butinyl-N-(2-chlor-6-methylphenyl)amino]imidazolin;

2-[N-2-Butinyl-N-(2-trifluormethyl-6-chlorphenyl)-amino]imidazolin;

2-[N-(3-Phenyl-2-propin-1-yl)-N-(2-trifluormethyl-6-chlorphenyl)amino]imidazolin;

2-[N-2-Butinyl-N-(2,6-dichlor-4-methylphenyl)amino]-imidazolin;

2-[N-2-Butinyl-N-(2,3-dichlorphenyl)amino]imidazolin;

2-[N-2-Butinyl-N-(2-methoxy-5-trifluormethylphenyl)amino]-imidazolin;

2-[N-2-Butinyl-N-(2,6-dichlor-3-methylphenyl)amino]-imidazolin;

2-[N-2-Butinyl-N-(2,4-dichlorphenyl)amino]imidazolin;

2-[N-2-Butinyl-N-(2-chlor-4-cyclopropylphenyl)amino]-imidazolin;

2-[N-2-Butinyl-N-(3-fluor-4-methylphenyl)amino]imidazolin;

2-[N-(3-Phenyl-2-propin-1-yl)-N-(2-chlor-6-methylphenyl)-amino]imidazolin;

2-[N-(3-Phenyl-2-propin-1-yl)-N-(2-trifluormethyl-6-fluorphenyl)amino]imidazolin;

2-[N-(3-Phenyl-2-propin-1-yl)-N-(2,6-dichlor-4-methyl)-amino]imidazolin;

2-[N-(3-Phenyl-2-propin-1-yl)-N-(2,6-dichlorphenyl)amino]-imidazolin;

2-[N-(3-Brom-2-propin-1-yl)-N-(2,6-dichlorphenyl)amino]-imidazolin;

2-[N-(3-Pentin-1-yl)-N-(2,6-dichlorphenyl)amino]imidazolin;

2-[N-(3-Pentin-1-yl)-N-(2-trifluormethyl-6-chlorphenyl)-amino]imidazolin;

2-[N-(3-Pentin-1-yl)-N-(2-trifluormethyl-6-fluorphenyl)-amino]imidazolin.

Die Herstellung von N-substituierten N-Phenylaminoimidazolinen kann nach verschiedenen Verfahren erfolgen, so wie es beispielsweise in der DE-OS 19 58 2o1 beschrieben ist.
Die erfindungsgemäßen Imidazoline der allgemeinen Formel I werden bevorzugt nach folgenden Verfahren hergestellt:

a) Substituierte Phenyliminoimidazolidine der allgemeinen Formel II

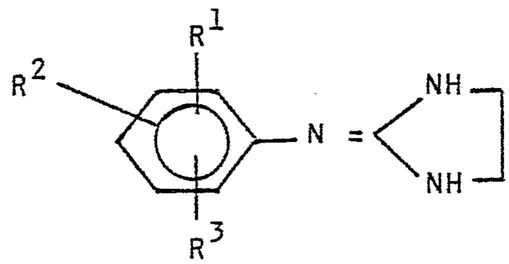

in der $R^1$, $R^2$ und $R^3$ wie oben angegeben definiert sind, werden mit einem Alkinylderivat der allgemeinen Formel III

$$R^4 - C \equiv C - (CH_2)_n - A$$

in der $R^4$ und n wie oben angegeben sind und A eine leicht abspaltbare Austrittsgruppe, wie z.B. Halogen oder eine Arylsulfonatgruppe, darstellt, umgesetzt.

Die Umsetzung der beiden Komponenten mit den allgemeinen Formeln II und III erfolgt zweckmäßigerweise in einem inerten organischen Lösungsmittel wie beispielsweise Acetonitril, Tetrahydrofuran oder Alkoholen, wie z.B. Ethanol und kann mit oder ohne den Zusatz von Base, z.B. Natriumcarbonat, durchgeführt werden.
Die Reaktionstemperaturen liegen zwischen 2o und 12o°C, vorzugsweise bei 8o°C.

Die als Ausgangsverbindungen verwendeten Phenyliminoimidazolidinderivate der allgemeinne Formel II sind aus zahlreichen Veröffentlichungen bekannt, so z.B. aus der DE-OS 23 16 377.

b) Weitere Verfahren zur Herstellung von Verbindungen der allgemeinen Formel I ist die Umsetzung von Verbindungen der allgemeinen Formel IV

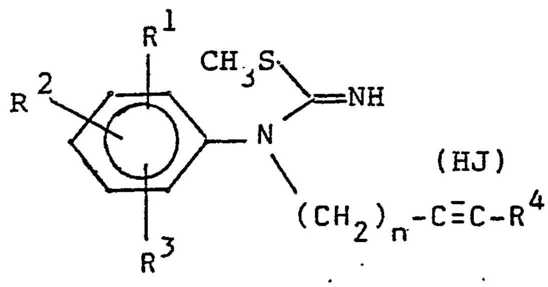

(HJ)

oder deren Säureadditionssalze mit Ethylendiamin, oder

die Umsetzung von Verbindungen der allgemeinen Formel V

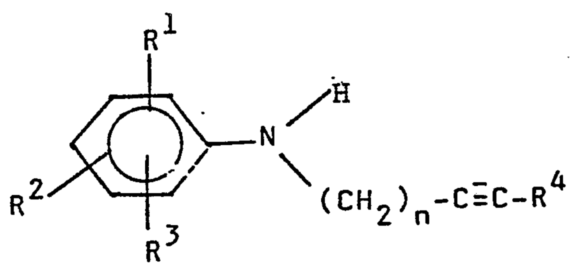

mit einer der folgenden Verbindungen

α) 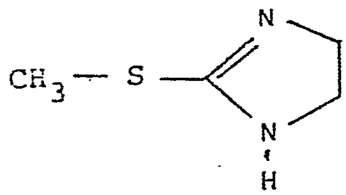

oder

ß)

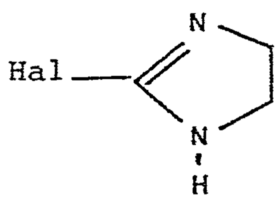

oder

γ)

1) 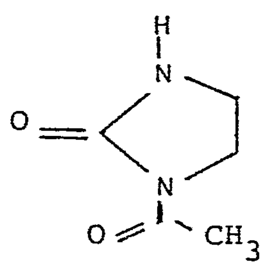 / $POCl_3$

2) $CH_3OH$ oder NaOH/Ethanol.

$R^1$ - $R^4$ und n haben die zuvor definierte Bedeutung,
Hal bedeutet ein Halogenatom, bevorzugt Chlor.

Die Umsetzung der einzelnen Komponenten erfolgt zweckmäßigerweise in inerten organischen Lösungsmitteln - wie z.B. halogenierten Kohlenwasserstoffen, Alkoholen, Acetonitril oder Tetrahydrofuran bei erhöhten Temperaturen, vorzugsweise bei der Rückflußtemperatur des Reaktionsgemisches.

Die Synthese der Ausgangsverbindungen ist in den Syntheseschemata I und II dargestellt und erfolgt nach allgemein bekannten Analogieverfahren.

Syntheseschema I



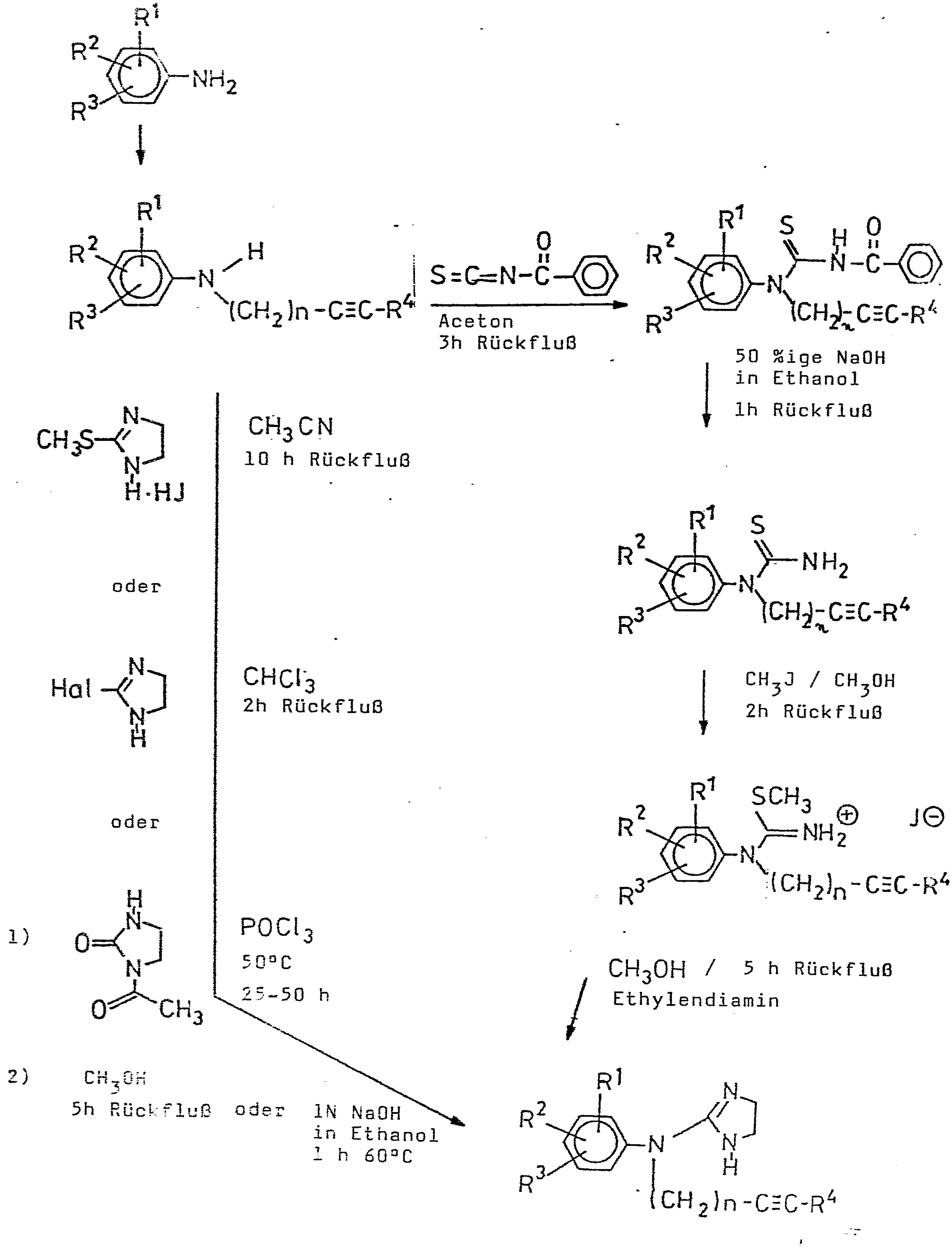

Syntheseschema II



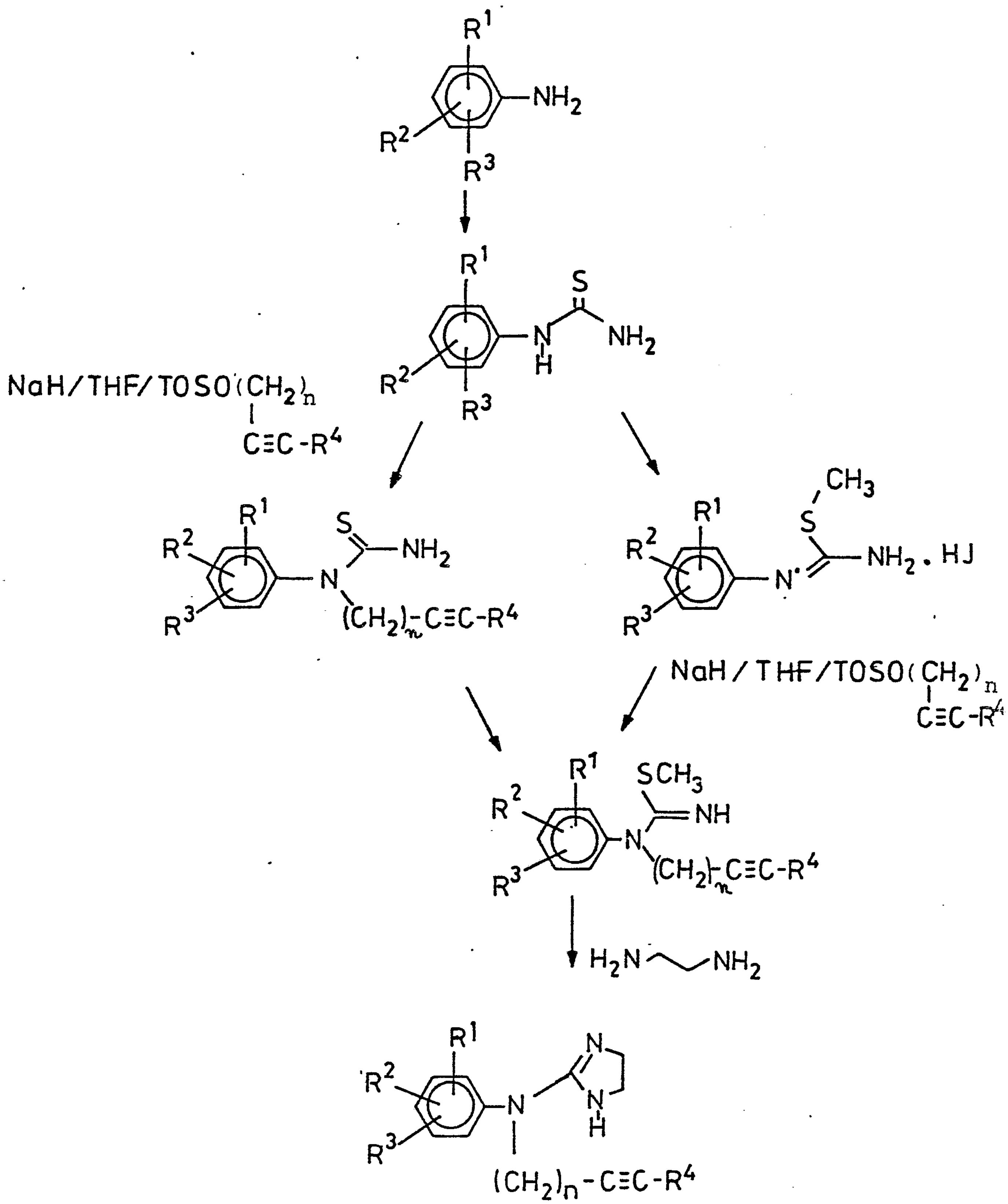


TOS = Toluolsulfonylgruppe

THF = Tetrahydrofuran

Die erfindungsgemäßen 2-(N-Alkinyl-N-phenylamino)-imidazolinderivate der allgemeinen Formel I können auf übliche Weise in ihre physiologisch verträglichen Säureadditionssalze überführt werden. Zur Salzbildung geeignete Säuren sind z.B. Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Valeriansäure, Capronsäure, Oxalsäure, Malonsäure, Bernsteinsäure, Glutarsäure, Maleinsäure, Fumarsäure, Milchsäure, Weinsäure, Zitronensäure, Äpfelsäure, Benzoesäure, p-Hydroxybenzoesäure, Phthalsäure, Zimtsäure, Salicylsäure, Ascorbinsäure, 8-Chlortheophyllin und dergl.

Die erfindungsgemäßen Verbindungen sowie deren Säureadditionssalze haben wertvolle pharmakologische Eigenschaften. Sie senken die Herzfrequenz durch direkten Angriff am Sinusknoten (spezifische Bradycardie). Sie empfehlen sich vor allem in der Indikation der Koronaren Herzkrankheit. Die unerwünschte metabolische Bildung von Phenyliminoimidazolidin-Derivaten (wie z.B. Clonidin) ist bei den erfindungsgemäßen Substanzen überraschenderweise deutlich zurückgedrängt, so daß bei der Therapie nicht mehr mit relevanten "Blutspiegelwerten des Metaboliten" zu rechnen ist.

Die bei bestimmten Phenylamino imidazolin-Derivate bekannte analgetische Wirkung ist deutlich gegenüber der spezifisch bradycarden Wirkung in den Hintergrund getreten. Die analgetische Wirkung wird bei der Therapie der Koronaren Herzkrankheit als ungünstig angesehen.

Die neuen Verbindungen können allein oder gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemittel, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragées durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die den Wirkstoff, beziehungsweise die Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol, beziehungsweise dessen Derivaten, herstellen.

Zum Zweck der transdermalen Applikation können die erfindungsgemäßen Wirkstoffe in entsprechend geeignete Träger (Pflaster), beispielsweise aus Polyacrylaten, eingearbeitet werden. Geeignete Adjuvantien können gegebenenfalls eingesetzt werden, um die Freigaberate zu erhöhen.

Die nachfolgenden Beispiele illustrieren die vorliegende Erfindung, ohne sie jedoch in ihrem Umfang zu beschränken.

Beispiel 1

6,9 g 2-(2,6-Dichlorphenyl-imino)imidazolidin (o,o3 Mol) werden in 6o ml wasserfreiem Acetonitril gelöst, mit 6,7 g 2-Butin-1-yl-toluolsulfonat (o,o3 Mol) versetzt und 5 Stunden unter Rückfluß gekocht. Nach dem Abkühlen wird im Vakuum zur Trockne eingeengt, wobei als Rückstand eine glasartige Masse resultiert, die in 1n HCl aufgenommen wird. Es wird mittels 1 n NaOH neutralisiert, und durch 4-maliges Extrahieren mit Ether trennt man Nebenprodukte ab. Die wässerige Phase wird in 1 n NaOH alkalisiert, was durch Stehen bei 0°C zur Abscheidung eines Öls führt, das später kristallisiert. Die Kristalle werden abgesaugt, mit Wasser gewaschen und getrocknet, wodurch man 5,7 g 2-[N-2-Butinyl-N-(2,6-dichlorphenyl)-amino]imidazolin erhält.
(Ausbeute: 67 %).

Die erhaltene Base läßt sich im ethanolisch-wäßrigen Milieu mittels konz. HBr in das Hydrobromid überführen.
Fp.: 2o6-2o7°C.

Beispiel 2

In Analogie zu Beispiel 1 werden 3,7 g 2-(2-Trifluormethoxy-5-fluor-phenylimino)-imidazolidin umgesetzt zum 2-[N-2-Butinyl-N-(2-trifluormethoxy-5-fluorphenyl)amino]imidazolin. Dabei erhält man 2,6 g (Ausbeute 58 %) vom Schmelzpunkt 95 - 1o6°C.

Beispiel 3

Analog zu Beispiel 1 werden 7,17 g 2-(2-Trifluormethyl-6-fluorphenylimino)imidazolidin butinyliert zum 2-[N-2-Butinyl-N-(2-trifluormethyl-6-fluorphenyl)amino]imidazolin. Man gewinnt 4,3 g (Ausbeute 49 %) in Form weißer Kristalle vom Fp. 1o5 - 1o6°C.

Beispiel 4

Durch analoges Vorgehen wie in Beispiel 1 überführt man 6,1 g 2-(2-Chlor-6-methyl-phenylimino)imidazolidin in das entsprechende 2-[N-2-Butinyl-N-(2-chlor-6-methylphenyl)-amino]imidazolin. Es werden 4,1 g (Ausbeute 54 %) in Form weißer Kristalle vom Fp. 1oo - 1o2°C gewonnen.

Beispiel 5

Analog zu Beispiel 1 setzt man 4,5 g 2-(2-Trifluormethyl-6-chlor-phenylimino)imidazolidin um zum 2-[N-2-Butinyl-N-(2-trifluormethyl-6-chlorphenyl)amino]imidazolin, von dem man 2,7 g (Ausbeute 5o %) in Form der freien Base erhält;
Fp. 1o7 - 1o9°C.

Beispiel 6

Analog zu Beispiel 1 werden 4,88 g 2-(2,6-dichlor-4-methyl-phenylimino)imidazolidin umgesetzt zum 2-[N-2-Butinyl-N-(2,6-dichlor-4-methylphenyl)amino]imidazolin. Es werden 3,3 g als Hydrochlorid (Ausbeute 49 %) in Form weißer Kristalle isoliert;
Fp. 2o8 - 211°C.

Beispiel 7

In Analogie zu Beispiel 1 werden 5,32 g 2-(2,3-dichlor-phenylimino)imidazolidin umgewandelt in 2-[N-2-Butinyl-N-(2,3-dichlorphenyl)amino]imidazolin. Man erhält somit 3,2 g (Ausbeute 49 %) als freie Base vom Fp. 94 - 95°C.

Beispiel 8

Durch analoge Vorgehensweise wie in Beispiel 1 werden 5 g 2-(2-Methoxy-5-trifluormethylphenylimino)imidazolidin umgesetzt zum 2-[N-2-Butinyl-N-(2-methoxy-5-trifluormethylphenyl)amino]imidazolin. Es werden 1,9 g (Ausbeute 31 %) als weiße Festsubstanz vom Fp. 1o9 - 111°C erhalten.

Beispiel 9

Analog zu Beispie 1 setzt man 5 g 2-(2,6-Dichlor-3-methyl-phenylimino)imidazolidin um zum 2-[N-2-Butinyl-N-(2,6-dichlor-3-methylphenyl)amino]imidazolin. Es lassen sich 2,3 g als Hydrobromid (Ausbeute 3o %) in Form weißer Kristalle vom Fp. 2o4 - 2o6°C isolieren.

Beispiel 1o

1o,48 g 2-(2-Chlor-6-methylphenylimino)imidazolidin (o,o5 Mol) werden in 75 ml wasserfreiem Acetonitril gelöst, mit 15 g 3-Phenyl-2-propin-1-yl-toluolsulfonat (o,o524 Mol) versetzt und über zwei Stunden zum Sieden erhitzt. Die Reaktionsmischung wird einrotiert und der ölige Rückstand in verdünntem NaOH aufgenommen und mit Essigester extrahiert. Die Essigesterphase wird eingeengt und der Rückstand in 2 n Salzsäure gelöst. Man alkalisiert mittels verdünntem NaOH auf pH 8, extrahiert mehrmals mit Ether, alkalisiert weiter bis pH 1o und extrahiert wiederum mit Ether. Letztere Etherphase wird über $MgSO_4$ getrocknet und eingeengt, wobei man 2-[N-(3-Phenyl-2-propin-1-yl)-N-(2-chlor-6-methylphenyl)amino]imidazolin erhält, das ins Hydrochlorid überführt wird. Es resultieren 16 g des Hydrochlorids (Ausbeute 7o%) als weißes, kristallines Produkt, dessen Fp. 192 - 194°C beträgt.

Beispiel 11

Auf analoge Weise wie in Beispiel lo werden 6,18 g 2-(2-Trifluormethyl-6-fluorphenylimino)imidazolidin umgewandelt zum 2-[N-(3-Phenyl-2-propin-1-yl)-N-(2-trifluormethyl-6-fluorphenyl)amino]imidazolin. Man erhält 7,65 g (Ausbeute 84 %) als hellgelbes Öl;
$R_f$ = o,54 im Fließmittel Toluol : Dioxan : EtOH : Ammoniak = lo : 8 : 3 : 1.

Beispiel 12

Analog zu Beispiel lo setzt man 4,5 g 2-(2-Trifluormethyl-6-chlorphenylimino)imidazolidin um zum 2-[N-(3-Phenyl-2-propin-1-yl)-N-(2-trifluormethyl-6-chlorphenyl)amino]-imidazolin. Man erhält 2,3 g (Ausbeute 36 %) in Form weißer Kristalle vom Fp. 116 - 117°C.

Beispiel 13

Analog zu Beispiel lo werden 4 g 2-(2,6-Dichlor-4-methyl-phenylimino)imidazolidin alkinyliert zum 2-[N-(3-Phenyl-2-propin-1-yl)-N-(2,6-dichlor-4-methylphenyl)amino]imidazolin, das in Form des Hydrochlorids isoliert wird. Es resultieren 3,52 g (Ausbeute 54 %) als weiße Kristalle vom Fp. 116 - 118°C.

Beispiel 14

Analog zu Beispiel lo werden 2o g 2-(2,6-Dichlorphenyl-imino)imidazolidin umgesetzt zum 2-[N-(3-Phenyl-2-propin-1-yl)-N-(2,6-dichlorphenyl)amino]imidazolin. Man gewinnt dabei 21,72 g (Ausbeute 65 %) in Form weißer Kristalle vom Fp. 126 - 127°C.

Beispiel 15

2o g 2-(2,6-Dichlorphenylimino)imidazolidin (o,o87 Mol) werden zusammen mit 3o g 3-Pentin-1-yl-toluolsulfonat

(77 %ig; o,o96 Mol) und 25o ml wasserfreiem Acetonitril 3o Stunden am Rückfluß gekocht. Die Reaktionsmischung wird eingeengt, das erhaltene Öl mit 15o ml 2 n NaOH und 3oo ml Ether versetzt und geschüttelt. Die wäßrige Phase wird abgetrennt und verworfen. Die Etherphase wird mit 15o ml 1 n HCl extrahiert, die salzsaure wäßrige Phase mittels verdünntem NaOH auf pH 9 gestellt, ausgeethert, mit NaOH stark alkalisch gestellt und mit Ether extrahiert. Letztere Etherphase trocknet man über $MgSO_4$, engt ein und erhält somit 9,5 g 2-[N-(3-Pentin-1-yl)-N-(2,6-dichlorphenyl)amino]imidazolin (Ausbeute 37 %) in Form weißer Kristalle vom Fp. 124 - 125°C.

Beispiel 16

Analog zu Beispiel 15 werden 4,5 g 2-(2-Trifluormethyl-6-chlorphenylimino)imidazolidin (o,o17 Mol) umgesetzt zu 2-[N-(3-Pentin-1-yl)-N-(2-trifluormethyl-6-chlorphenyl)-amino]imidazolin. Man erhält 1,9 g (Ausbeute 34 %) in Form weißer Kristalle vom Fp. 1o9 - 11o°C.

Beispiel 17

In Analogie zu Beispiel 15 überführt man 15 g 2-(2-Trifluormethyl-6-fluorphenylimino)imidazolidin (o,o61 Mol) in das 2-[N-(3-Pentin-1-yl)-N-(2-trifluormethyl-6-fluorphenyl)-amino]imidazolin. Man gewinnt 1o,8 g (Ausbeute 57%) als weiß-kristalline Substanz vom Fp. 86 - 89°C.

Beispiel 18

Analog zu Beispiel 1 werden 25 g 2-(2,4-Dichlorphenyl-imino)-imidazolidin umgesetzt zum 2-[N-2-Butinyl-N-(2,4-dichlorphenyl)-amino]imidazolin. Dabei erhält man 3,8 g als Hydrobromid (Ausbeute 18 %) in Form bräunlicher Kristalle; Fp. 156 - 158°C.

Beispiel 19

8 g 2-(3-Fluor-4-methylphenyl-imino)imidazolidin werden wie in Beispiel 1 butinyliert zum 2-[N-2-Butinyl-N-(3-fluor-4-methylphenyl)-amino]imidazolin. Die erhaltene Base wird in das Hydrobromid überführt, von dem 2 g (Ausbeute 15 %) als weiße Kristalle erhalten werden; Fp. 186 - 193°C.

Beispiel 20

8 g 2-(2-Chlor-4-cyclopropylphenyl-imino)-imidazolidin werden in Analogie zu Beispiel 1 umgesetzt zum 2-[N-2-Butinyl-N-(2-chlor-4-cyclopropylphenyl)-amino]imidazolin. Es werden 3,9 g in Form des Hydrochlorids (Ausbeute 40 %) als weiße Kristalle erhalten; Fp. 231 - 234°C.

Beispiel 21

12,13 g 2-(2,6-Dichlorphenyl-imino)imidazolidin (0,053 Mol) und 12,05 g 1,3-Dibrompropin (Gehalt 95 %; 0,058 Mol) werden in ca. 100 ml wasserfreiem Acetonitril aufgenommen und 20 Stunden bei Raumtemperatur gerührt. Es wird abgesaugt und der erhaltene gelbe Niederschlag (7,9 g) mittels einer Kieselgelsäule und Methanol: Essigester 1:1 chromatographisch getrennt. Von den drei aufgefangenen Fraktionen enthält die zweite das gewünschte 2-[N-(3-Brom-2-propinyl)-N-(2,6-dichlorphenyl)-amino]imidazolin.
Man erhält so 1,25 g der freien Base (Ausbeute 7,7 %) in Form gelber Kristalle; Fp. ab 145°C (Zersetzung).

Beispiel A: Tabletten

| | |
|---|---|
| 2-[N-2-Butinyl-N-(2,6-dichlorphenyl)-amino]imidazolin-hydrobromid | 1o mg |
| Milchzucker | 65 mg |
| Maisstärke | 125 mg |
| sek. Calciumphosphat | 4o mg |
| lösliche Stärke | 3 mg |
| Magnesiumstearat | 3 mg |
| kolloidale Kieselsäure | 4 mg |
| insgesamt | 25o mg |

Herstellung:
Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Drageekernen von 25o mg Gewicht verpreßt, die dann in üblicher Weise mit Hilfe von Zucker, Talkum und Gummi arabicum dragiert werden.

Beispiel B: Ampullen

| | | |
|---|---|---|
| 2-[N-2-Butinyl-N-(2,6-dichlorphenyl)amino]imidazolin-hydrobromid | | 1,o mg |
| Natriumchlorid | | 18,o mg |
| dest. Wasser | ad | 2,o ml |

Herstellung:
Wirkstoff und Natriumchlorid werden in Wasser gelöst und unter Stickstoff in Glasampullen abgefüllt.

Beispiel C: Tropfen

| | | |
|---|---|---|
| 2-[N-2-Butinyl-N-(2,6-dichlorphenyl)amino]imidazolin-hydrobromid | | o,o2 g |
| p-Hydroxybenzoesäuremethylester | | o,o7 g |
| p-Hydroxybenzoesäurepropylester | | o,o3 g |
| entmineralisiertes Wasser | ad | loo ml |

Beispiel D: Injektionslösung

| | | |
|---|---|---|
| 2-[N-2-Butinyl-N-(2,6-dichlorphenyl)amino]imidazolin-hydrobromid | | 1,5 Teile |
| Natriumsalz der Äthylendiamintetraessigsäure | | o,2 Teile |
| dest. Wasser | ad | loo,o Teile |

Herstellung:

Der Wirkstoff und das Natriumsalz der Äthylendiamintetraessigsäure werden in genügend Wasser gelöst und mit Wasser auf das gewünschte Volumen aufgefüllt. Die Lösung wird von suspendierten Partikeln filtriert und in 2 ml Ampullen unter aseptischen Bedingungen abgefüllt. Zuletzt werden die Ampullen sterilisiert und verschlossen. Jede Ampulle enthält 2o mg Wirkstoff.

Patentansprüche

1. Substituierte 2-(N-Alkinyl-N-phenylamino)imidazoline der allgemeinen Formel

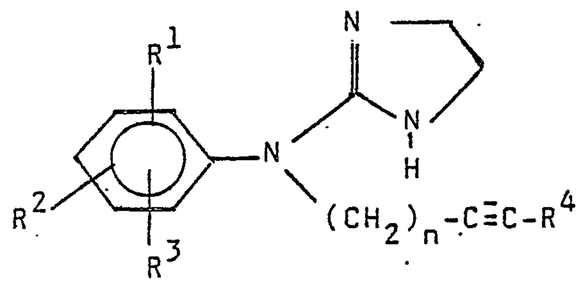

I,

worin
$R^1$, $R^2$ und $R^3$ gleich oder verschieden sein können und Wasserstoff, Halogen, eine verzweigte oder unverzweigte niedere Alkyl-, Halogenalkyl-, Alkoxy- oder Halogenalkoxygruppe, eine Cycloalkylgruppe,
$R^4$ Halogen, eine verzweigte oder unverzweigte niedere Alkylgruppe, eine Arylgruppe, gegebenenfalls mit Halogen oder wenigstens einer verzweigten oder unverzweigten niederen Alkyl-, Halogenalkyl-, Alkoxy-, Halogenalkoxygruppe substituiert, oder eine Aralkylgruppe, gegebenenfalls mit Halogen oder wenigstens einer verzweigten oder unverzweigten niederen Alkyl-, Halogenalkyl-, Alkoxy-, Halogenalkoxygruppe substituiert und
n die Zahlen 1, 2 oder 3 bedeuten können sowie deren Säureadditionssalze

2. Verbindungen nach Anspruch 1, dadurch gekennzeichnet, daß $R^1$, $R^2$ und $R^3$ gleich oder verschieden sein können und Wasserstoff, Halogen, bevorzugt Fluor oder Chlor, eine verzweigte oder unverzweigte Alkyl-, Halogenalkyl-, Alkoxy- oder Halogenalkoxygruppe mit 1 bis 4 Kohlenstoffatomen, eine Cyclopropylgruppe;

$R^4$ Halogen, bevorzugt Chlor oder Brom, eine verzweigte oder unverzweigte Alkylgruppe mit 1 bis 4 Kohlenstoffatomen, eine Phenylgruppe, gegebenenfalls mit Halogen oder wenigstens einer verzweigten oder unverzweigten Alkyl-, Halogenalkyl-, Alkoxy- oder Halogenalkoxygruppe mit 1 bis 4 Kohlenstoffatomen substituiert, oder eine Phenalkylgruppe mit 1 bis 4 Kohlenstoffatomen in der Alkylkette und gegebenenfalls mit wenigstens einem Halogen- oder Alkyl-, Halogenalkyl-, Alkoxy- oder Halogenalkoxysubstituenten in der Phenylgruppe und n die Zahlen 1, 2 oder 3 bedeuten sowie deren Säureadditionssalze.

3) Verbindungen nach Anspruch 1, dadurch gekennzeichnet, daß $R^1$, $R^2$ und $R^3$ gleich oder verschieden sein können, Wasserstoff, Fluor, Chlor, Brom, Methyl, Cyclopropyl, Trifluormethyl, Methoxy oder Trifluormethoxy,

$R^4$ Brom, Methyl oder Phenyl und n 1 oder 2 bedeuten, sowie deren Säureadditionssalze.

4) 2-[N-2-Butinyl-N-(2,6-dichlorphenyl)amino]imidazolin.

5) Verfahren zur Herstellung von 2-(N-Alkinyl-N-phenylamino)-imidazolinen der allgemeinen Formel I

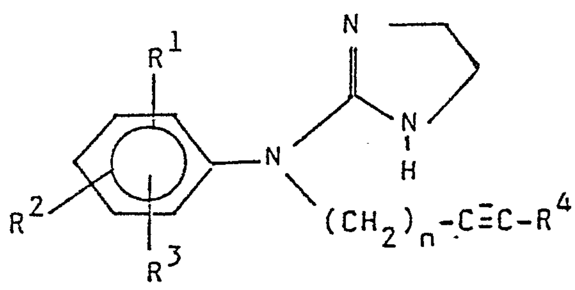

I,

worin
$R^1$, $R^2$ und $R^3$ gleich oder verschieden sein können und Wasserstoff, Halogen, eine verzweigte oder unverzweigte niedere Alkyl-, Halogenalkyl-, Alkoxy- oder Halogenalkoxygruppe, eine Cycloalkylgruppe,
$R^4$ Halogen, eine verzweigte oder unverzweigte niedere Alkylgruppe, eine Arylgruppe, gegebenenfalls mit Halogen oder wenigstens einer verzweigten oder unverzweigten niederen Alkyl-, Halogenalkyl-, Alkoxy-, Halogenalkoxygruppe substituiert, oder eine Aralkylgruppe, gegebenenfalls mit Halogen oder wenigstens einer verzweigten oder unverzweigten niederen Alkyl-, Halogenalkyl-, Alkoxy-, Halogenalkoxygruppe substituiert und
n die Zahlen 1, 2 oder 3 bedeuten können,
dadurch gekennzeichnet, daß man

a) eine Verbindung der allgemeinen Formel

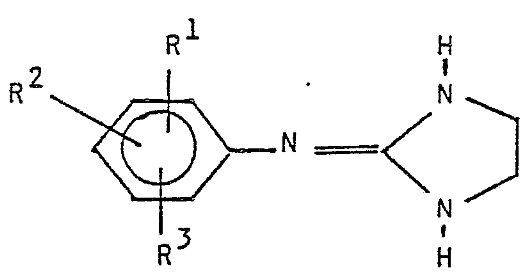

II

in der $R^1$, $R^2$ und $R^3$ wie zuvor angegeben definiert sind mit einem Alkinylderivat der allgemeinen Formel

$$R^4 - C \equiv C - (CH_2)_n - A \qquad III$$

in der $R^4$ und n wie zuvor angegeben definiert sind und A eine leicht abspaltbare Austrittsgruppe, wie z.B. Halogen-oder eine Arylsulfonatgruppe, darstellt, umsetzt,

oder

b) eine Verbindung der allgemeinen Formel IV

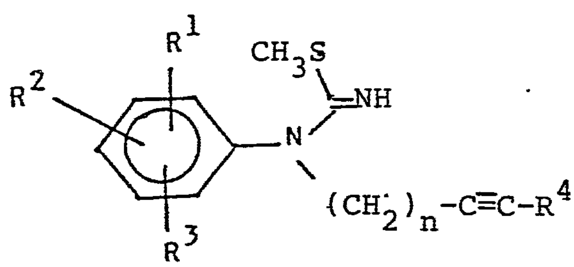

oder deren Säureadditionssalz worin $R^1$, $R^2$, $R^3$, $R^4$ und n die oben genannte Bedeutung haben mit Ethylendiamin umsetzt,

oder

c) eine Verbindung der allgemeinen Formel V

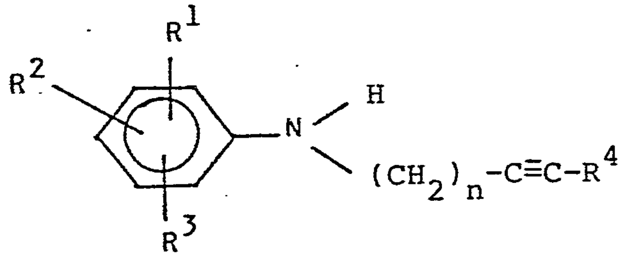

worin $R^1$, $R^2$, $R^3$, $R^4$ und n die oben genannte Bedeutung haben,

mit einer der folgenden Verbindungen

α)

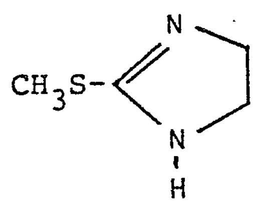

oder

ß)

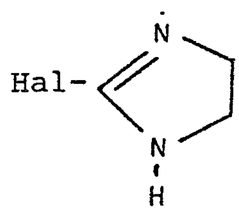

oder

γ) 1)

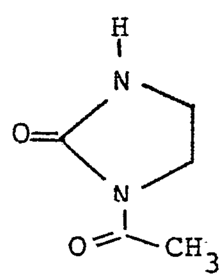

/ $POCl_3$

2) $CH_3OH$ oder NaOH/Ethanol

umsetzt und

die nach den Verfahren a - c erhaltenen Verbindungen gegebenenfalls in ihre Säureadditionssalze überführt.

6) Pharmazeutische Zubereitungen, dadurch gekennzeichnet, daß sie als Wirkstoff eine oder mehrere Verbindungen nach Anspruch I enthalten.

7) Verfahren zur Herstellung von pharmazeutischen Zubereitungen nach Anspruch 6, dadurch gekennzeichnet, daß man eine oder mehrere Verbindungen nach Anspruch 1 mit üblichen Hilfs- oder Trägerstoffen, Spreng- oder Schmiermitteln oder Substanzen zur Erzielung einer Depotwirkung zu pharmazeutischen Zubereitungen verarbeitet.

8) Verbindungen nach Anspruch 1 zur Verwendung für die Therapie der koronaren Herzkrankheit.

0202461

Europäisches Patentamt

# EUROPÄISCHER RECHERCHENBERICHT

Nummer der Anmeldung

EP 86105042.5

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 029 505 (BOEHRINGER) * Zusammenfassung; Seite 9, Beispiel 12 * -- | 1-3,5-8 | C 07 D 233/50 A 61 K 31/415 |
| A | CH - A5 - 621 116 (BOEHRINGER) * Anspruch 1; Seite 3, linke Spalte, Zeilen 59-64 * -- | 1-3,5-8 | |
| A | US - A - 4 277 487 (STAHLE) * Zusammenfassung * ---- | 1-3,5-8 | |
| | | | RECHERCHIERTE SACHGEBIETE (Int. Cl.4) |
| | | | C 07 D 233/00 |

Der vorliegende Recherchenbericht wurde für alle Patentansprüche erstellt.

| Recherchenort | Abschlußdatum der Recherche | Prüfer |
|---|---|---|
| WIEN | 26-08-1986 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument